# EUROPÄISCHE PATENTSCHRIFT

(11) **EP 2 934 753 B1**
(45) Veröffentlichungstag und Bekanntmachung des Hinweises auf die Patenterteilung: **04.05.2022**
(21) Anmeldenummer: 13821812.8
(22) Anmeldetag: 18.12.2013
(51) Int. Cl.: B01L 3/02, A61M 5/315

(54) **DOSIERPIPETTE**
DOSING PIPETTE
PIPETTE POUR DOSAGE

(30) Priorität: 18.12.2012 DE 102012112498
(43) Veröffentlichungstag der Anmeldung: 28.10.2015
(73) Patentinhaber: BALDA Medical GmbH, 32549 Bad Oeynhausen (DE)
(72) Erfinder: FUCHS, Karl-Heinz, 78315 Radolfzell (DE)
(74) Vertreter: Isarpatent
(86) Internationale Anmeldenummer: PCT/EP2013/077210
(87) Internationale Veröffentlichungsnummer: WO 2014/096081

(56) Entgegenhaltungen:
- EP-A- 0 087 731
- WO-A1-00/18453
- WO-A1-2004/054720
- DE-A1- 3 604 826
- US-A1- 2004 162 528
- US-A1- 2012 283 653
- US-A1- 2012 283 657

## Beschreibung

### Technisches Gebiet

Die Erfindung betrifft ein Dosierpipettensystem zum Ausbringen eines Mediums nach dem Oberbegriff des Anspruchs 1. Bevorzugte Ausführungsbeispiele sind Gegenstand der Unteransprüche

### Stand der Technik

Aus dem Stand der Technik sind verschiedene Pipetten und Entnahmesysteme für verschiedene Medien, insbesondere für flüssige Medien, bekannt. Diese kommen sowohl in vielen Fällen des täglichen Lebens als auch bei industriellen Anwendungen zum Einsatz, um Flüssigkeiten oder andere Medien aus einem Behälter zu entnehmen.

Besonders häufig, kommen die bekannten Pipettensysteme im Bereich der Medizin zum Einsatz. Bei diesen Pipettensystemen wird das flüssige Medium meist über einen handbetätigten Kolben in das Pipettensystem aufgezogen. Hierbei muss sich der Anwender an der Skalierung auf dem Pipettenbehälter orientieren. D. h., dass der Benutzer den Ansaugkolben des Pipettensystems innerhalb der Dosierskalierung beliebig bewegen kann.

Dadurch ist es häufig der Fall, dass eine Uber- oder Unterdosierung vorliegt, da der Anwender auf Grund von Ablesefehlern oder Ableseungenauigkeiten den Ansaugkolben über oder unter die gewünschte Skalierung zieht.

Dies bedeutet weiterhin, dass die Dosierung nicht reproduzierbar ist, da es bei jedem Aufziehen zu Schwankungen kommen kann. Vor allem im medizinischen Bereich kann eine Über- oder Unterdosierung negative Auswirkungen haben. So kann eine Überdosierung gesundheitsschädlich sein, während eine Unterdosierung den Heilungsverlauf verzögern kann.

Ein weiterer Nachteil von Pipettensystemen aus dem Stand der Technik ist, dass beim Aufziehen des Mediums häufig Luftblasen im Bereich des Dosiervolumens im Pipettensystem entstehen. Diese Luftblasen können dazu führen, dass die aufgezogene Menge des Mediums nicht der Menge auf der Dosierskala entspricht. Weiterhin können Lufteinschlüsse vor allem im medizinischen Bereich für den Patienten lebensbedrohlich sein.

Eine Dosiervorrichtung zum Ansaugen und Ausbringen eines fliessfähigen Mediums ist bspw. aus der WO 2004/054720 bekannt. Diese Dosiervorrichtung weist einen Einlass und Durchlass auf, die voneinander getrennt angeordnet sind. Zwischen Einlass und Durchlass ist dabei eine Dosier- und Verdrängungskammer vorgesehen. Die Dosiervorrichtung ist jedoch ein bleibender Bestandteil des Behälters aus dem das Medium entnommen wird und kann von diesem nicht getrennt gehandhabt werden.

Eine weitere Dosiervorrichtung ist aus der WO 00/18453 bekannt. Bei dieser kann die gewünschte Menge über ein Rad, das sich an einem Behälterverschluss befindet, eingestellt werden. Jedoch ist auch diese Dosiervorrichtung nur in Kombination mit einem zugehörigen Behälter verwendbar.

Weitere Dosiervorrichtungen sind aus den Druckschriften US 2012/0283653 A1, US 2012/0283657 A1, US 2004/0162528 A1 und EP 0 087 731 A1 bekannt.

### Aufgabe der Erfindung

Die Aufgabe der Erfindung ist es, ein dosiertes Entnehmen eines Mediums, insbesondere eines flüssigen Mediums, aus einem Behälter zu ermöglichen. Insbesondere ist es Aufgabe der Erfindung, ein Pipettensystem zur Verfügung zu stellen, bei dem ein bestimmtes Dosiervolumen eingestellt werden kann und bei dem das Dosiervolumen reproduzierbar ist.

Weiterhin ist es Aufgabe der Erfindung, ein Pipettensystem zur Verfügung zu stellen, bei dem ein luftfreies und blasenfreies Ansaugen bzw. Aufziehen des Mediums ermöglicht wird. Auch ist es Aufgabe der Erfindung, ein System zur Verfügung zu stellen, bei dem bei großer Teilegleichheit ein breites Spektrum an Kundenanforderung bzgl. des Dosiervolumens abgedeckt werden kann.

Weiterhin soll ein Pipettensystem zur Verfügung gestellt werden, das leicht demontierbar und leicht reinigbar ist. Zudem soll das Pipettensystem so ausgebildet sein, dass es auch von ungeschulten bspw. älteren Menschen benutzt werden kann.

### Lösung der Aufgabe

Zur Lösung der Aufgabe führen die Merkmale nach dem Anspruch 1. Bevorzugte Ausführungsformen sind in den abhängigen Ansprüchen angegeben.

Eine Dosierpipette zum Ausbringen eines Mediums, insbesondere eines flüssigen Mediums, umfasst einen Dosierkolben und ein Gehäuse. Das Gehäuse umfasst eine Dosiergeometrie, wobei bei der erfindungsgemässen Dosierpipette die Dosiergeometrie von dem Gehäuse entkoppelbar ist.

Der Dosierkolben der Dosierpipette ist mehrteilig ausgebildet und besteht aus einem Kolben und einem Dosiereinsatz. Weiterhin ist der Dosierkolben in einem typischen Ausführungsbeispiel entnehmbar und/oder austauschbar. Dadurch kann der Dosierkolben einerseits auf Kundenanforderungen angepasst werden und andererseits zur Reinigung entnommen werden.

In einem typischen Ausführungsbeispiel weist der Kolben des Dosierkolbens zumindest eine Dosiernut auf. Vorzugsweise weist der Kolben mehrere Dosiernuten auf, die sich über den Umfang verteilt zumindest über einen Teilbereich der Länge des Kolbens erstrecken. Die Länge der Nuten kann über den Dosiereinsatz variiert werden, der als eine Art Hülse auf den Kolben aufgeschoben wird.

Um verschiedene Dosierwege bereitzustellen, welche wiederum für verschiedene Dosiervolumina sorgen, weist der Dosiereinsatz eine Dosiergeometrie auf. In einem typischen Ausführungsbeispiel handelt es sich bei der Dosiergeometrie um eine Stufengeometrie, welche so ausgeformt ist, dass die Länge der Dosiernuten des Kolbens auf die gewünschten Längen der Dosierwege beschränkt wird. Dies führt weiterhin dazu, dass die Dosierpipette bei grosser Teilegleichheit lediglich durch einen Austausch des Dosiereinsatzes in verschiedenen Ausführungsformen bzgl. der Dosiervolumina, hergestellt werden kann.

So kann bspw. in einem Ausführungsbeispiel der Dosierbereich der Dosierpipette von 1 ml bis 6 ml in 1 ml Schritten unterteilt werden, während der Dosierbereich in einem anderen Ausführungsbeispiel von 0,5 ml bis 5 ml in 0,5 ml Schritten unterteilt wird. Wie durch die Beispiele dargestellt, können die Dosiervolumina der Dosierpipette und somit die Dosierung des auszubringenden Mediums über den Dosiereinsatz eingestellt werden.

Weiterhin ist das Gehäuse der Dosierpipette zur Aufnahme verschiedener Dosierkolben geeignet, wobei der Dosiereinsatz des Dosierkolbens unterschiedlich ausgeformt ist Der Dosierkolben ist mit dem Gehäuse formschlüssig verbunden, da zumindest ein Anschlag in die Dosiernuten und/oder die Dosiergeometrie des Dosierkolbens greift, wenn die Dosierpipette montiert ist. Dadurch kann der Dosierkolben aber immer noch auf der Länge des eingestellten Dosierwegs aus dem Gehäuse herausgezogen werden und anschliessend wieder in diesen eingeschoben werden. Zur Begrenzung der Dosierwege weist die Dosiergeometrie zumindest einen Dosieranschlag auf, der mit dem festen Anschlag als begrenzendes Mittel in Wirkverbindung steht. Der Dosieranschlag begrenzt die Länge des Dosierwegs auf die gewünschte Länge und sorgt weiterhin dafür, dass der Dosierkolben nicht weiter als vorgesehen aus dem Gehäuse herausgezogen werden kann.

Der Anschlag ist in einem Ausführungsbeispiel in einen mehrteiligen Gehäuserand integriert. Ein Teil des Gehäuserands ist einstückig mit dem Gehäuse ausgebildet. Der Anschlag oder die Anschläge sind in einem typischen Ausführungsbeispiel einstückig mit einem Gehäuseflansch ausgebildet und weisen vorzugsweise die Form eines Nocken und oder Zapfen auf. Der Gehäuserand wird durch den Gehäuseflansch geschlossen, indem die Anschläge in dafür vorgesehene Ausnehmungen eingebracht werden. Dies geschieht, nachdem der Dosierkolben in das Gehäuse eingeführt wurde.

Um ein sicheres Schliessen des Gehäuserands zu gewährleisten, befinden sich die Ausnehmungen zur Aufnahme des Anschlags im Bereich des grössten Durchmessers des Gehäuses, der einem Tangentialpunkt entspricht, oder sind so am Gehäuse angebracht, dass die Anschläge des Gehäuseflanschs über den Tangentialpunkt geschoben werden müssen, um in die Ausnehmungen eingebracht werden zu können.

In einem typischen Ausführungsbeispiel handelt es sich bei dem Gehäuse und dem Gehäuserand zwar um zwei Teile, diese können aber um Kosten zu sparen als ein Bauteil, vorzugsweise als Spritzgussteil hergestellt werden. In diesen Fall ist der Gehäuseflansch nach der Teilefertigung im Bereich der Anschläge mit dem Gehäuse verbunden, wobei eine Perforation im Verbindungsbereich der beiden Teile dafür sorgt, dass der Gehäuseflansch einfach vom Gehäuse abgetrennt, insbesondere abgeschert werden kann, bevor dieser mit dem Gehäuse verbunden wird. Der Gehäuseflansch mit den Anschlägen kann dabei so ausgeführt sein, dass ein Auseinandernehmen der Dosierpipette nach der Montage verhindert wird.

In einem weiteren Ausführungsbeispiel ist der Anschlag in einen Verriegelungsring integriert. Der Verriegelungsring umfasst in einem typischen Ausführungsbeispiel mehr als die Hälfte des Umfangs des Gehäuses vorzugsweise etwa ¾ des Umfangs des Gehäuses und ist typischerweise unter dem in diesem Fall vorzugsweise einstückig ausgebildeten Gehäuserand angebracht.

Mit unter dem Gehäuserand wird dabei eine Lage beschrieben, die besagt, dass sich der Verriegelungsring auf einer proximalen Seite des Gehäuserands befindet. Durch die proximale Seite wird dabei die Seite beschrieben, die zum Körperzentrum hinzeigt und an deren Ende des Gehäuses sich eine Ausbringöffnung befindet durch die über den Dosierkolben ein Medium in Dosierpipette eingezogen und wieder ausgebracht werden kann.

Der Verriegelungsring kann in einem typischen Ausführungsbeispiel von der Dosierpipette heruntergeschoben werden, wodurch eine Entnahme des Dosierkolbens ermöglicht. Dadurch wird ermöglicht, dass die Einzelteile der Dosierpipette gereinigt werden können. Anschliessend wird die Dosierpipette durch Einsetzen des Dosierkolbens in das Gehäuse und anschliessendes Aufschieben des Verriegelungsrings wieder zusammengesetzt und kann erneut verwendet werden.

Der Anschlag und der Dosierkolben sind so ausgebildet, dass der Dosierkolben im Gehäuse zumindest teilweise drehbar gelagert ist. Vorzugsweise wird ein Verdrehen des Dosierkolbens lediglich in einem entleerten Zustand der Dosierpipette ermöglicht. Durch die Möglichkeit des Verdrehens des Dosierkolbens können mit der Dosierpipette verschiedene reproduzierbare Dosiervolumina eingestellt werden. Hierfür weist der Gehäuserand in einem typischen Ausführungsbeispiel auf der distalen Seite Markierungen auf, wobei durch distal eine in Gebrauchslage von einem Körperzentrum abgewandte Seite beschrieben wird. Weiterhin weist der Kolben auf der distalen Seite einen Kolbenflansch mit einer Nase auf, die beispielsweise als Pfeilgeometrie ausgebildet sein kann. Durch Verdrehen des Dosierkolbens im Gehäuse zeigt die Nase, abhängig von der durch Verdrehen eingestellten Position, auf eine der Markierungen auf dem Gehäuserand. Durch diese Angabe wird dem Anwender verdeutlicht, welches Dosiervolumina eingestellt ist. Weiterhin besteht die Möglichkeit, dass auf der distalen Seite des Kolbenflanschs ein Anwenderhinweis angebracht wird. Dieser kann aufgedruckt sein oder eingeprägt oder als Ausnehmung und/oder Erhebung bereits in der Teileherstellung bspw. beim Spritzgiessen eingebracht werden.

Um ein ungewolltes Verschieben des Dosiereinsatzes auf dem Kolben des Dosierkolbens zu verhindern, weist der Dosiereinsatz auf der proximalen Seite eine Ausbuchtung auf, die komplementär zu einem Rastelement ausgebildet ist, das sich im Bereich des proximalen Endes des Kolbens befindet. Die Ausbuchtungen und zugehörigen Rastelemente können verschiedenste Formgebungen aufweisen. In einem typischen Ausführungsbeispiel sind die Ausbuchtungen als kreis- und/oder ellipsenförmige Erhebungen ausgebildet.

Die Ausbuchtungen im Dosiereinsatz befinden sich vorzugsweise in einer Ausnehmung, die sich in einer Art Boden auf der proximalen Seite des Dosiereinsatzes befindet. Die Ausnehmung ist in einem typischen Ausführungsbeispiel komplementär zu dem Bereich des Kolbens ausgebildet in dem sich die Rastelemente befinden. Dadurch entsteht zwischen dem Kolben und dem Dosiereinsatz eine formschlüssige Verbindung, die ein ungewolltes Verschieben des Dosiereinsatzes auf dem Kolben verhindert.

Weiterhin weist der Dosierkolben eine Kolbenlippe und einen Dichtzapfen auf. In einem typischen Ausführungsbeispiel ist die Kolbenlippe dabei einstückig mit dem Dosiereinsatz des Dosierkolbens ausgebildet, wohingegen der Dichtzapfen einstückig mit dem Kolben ausgebildet ist.

Der Dichtzapfen sorgt dafür, dass eine saubere Applikation möglich ist und schliesst die Ausbringöffnung im Gehäuse im entleerten Zustand ab, ist somit in einem typischen Ausführungsbeispiel komplementär zu der Ausbringöffnung ausgebildet. Die Ausbringöffnung im Gehäuse ist einem typischen Ausführungsbeispiel als zylindrische und/oder konische Ausnehmung ausgebildet.

Die Kolbenlippe sorgt dafür, dass ein luft- und blasenfreies Ansaugen/Aufziehen und/oder Entleeren der Dosierpipette ermöglicht wird. Weiterhin sorgt diese Ausgestaltung dafür, dass nur eine sehr geringes Rest-/Todvolumen im entleerten Zustand der Dossierpipette vorhanden ist.

### Figurenbeschreibung

Weitere Vorteile, Merkmale und Einzelheiten der Erfindung ergeben sich aus der nachfolgenden Beschreibung bevorzugter Ausführungsbeispiele sowie anhand der Zeichnungen; diese zeigen in
- Figur 1: eine Schnittdarstellung längs durch eine erste erfindungsgemässe Dosierpipette in einer entleerten Gebrauchslage;
- Figur 2: eine Schnittdarstellung längs durch die Dosierpipette nach Figur 1 in aufgezogener Gebrauchslage;
- Figur 3: eine Detailansicht eines Bereichs der Dosierpipette in der sich ein Anschlag befindet;
- Figur 4: eine Draufsicht von einer distalen Seite auf ein Gehäuse mit einem mehrteiligen Gehäuserand;
- Figur 5: eine Schnittdarstellung quer durch das erste Gehäuse oberhalb des Anschlags;
- Figur 6: eine Seitenansicht auf ein Gehäuse nach Figur 4;
- Figur 7a-7c: verschiedne Ansichten eines Kolbens;
- Figur 8a-8b: verschiedene Ansichten einer Dosierhülse;
- Figur 9: eine Seitenansicht eines zusammengesetzten Dosierkolbens;
- Figur 10: eine Detailschnittdarstellung eines proximalen endes der Dosierpipette;
- Figur 11: eine weiteres Ausführungsbeispiel einer erfindungsgemässen Dosierpipette;
- Figur 12: einen Querschnitt durch die Dosierpipette nach Figur 11 mit einem ersten Ausführungsbeispiel des Verriegelungsrings; und
- Figur 13: einen Querschnitt durch die Dosierpipette nach Figur 11 mit einem weiteren Ausführungsbeispiel des Verriegelungsrings.

### Ausführungsbeispiel

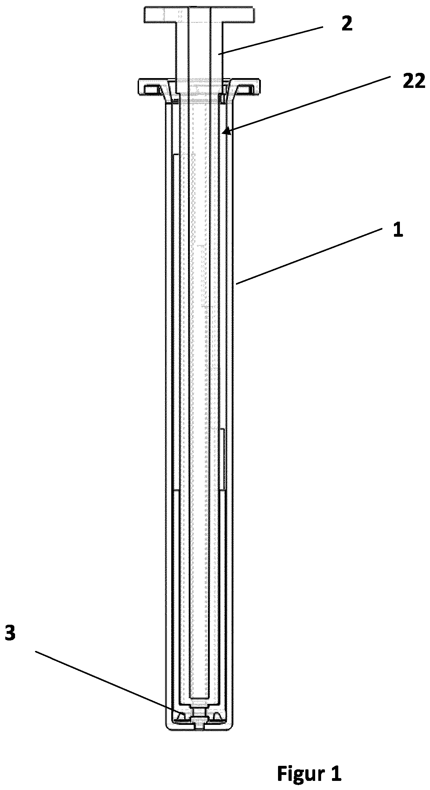

In Figur 1 ist eine Schnittdarstellung längs durch ein typisches Ausführungsbeispiel einer erfindungsgemässen Dosierpipette dargestellt, die zum Ausbringen und/oder zur Aufnahme eines Mediums geeignet ist..

Mit längs durch das System wird dabei eine Schnittebene beschrieben, die sich von einem proximalen Ende der Dosierpipette zu einem distalen Ende der Dosierpipette erstreckt. Durch das proximale Ende wird ein, einem Körper zugewandte Ende der Dosierpipette beschrieben, in dem sich eine Ausbringöffnung 11 befindet. Durch das distale Ende wird ein dem Körper abgewandtes Ende der Dosierpipette beschrieben, das sich auf der gegenüberliegenden Seite des proximalen Endes mit der Ausbringöffnung 11 befindet.

Die Dosierpipette besteht in einem typischen Ausführungsbeispiel aus einem Gehäuse 1 und einem Dosierkolben 22. Der Dosierkolben 22 besteht dabei in einem typischen Ausführungsbeispiel aus einem Kolben 2 und einem Dosiereinsatz 3.

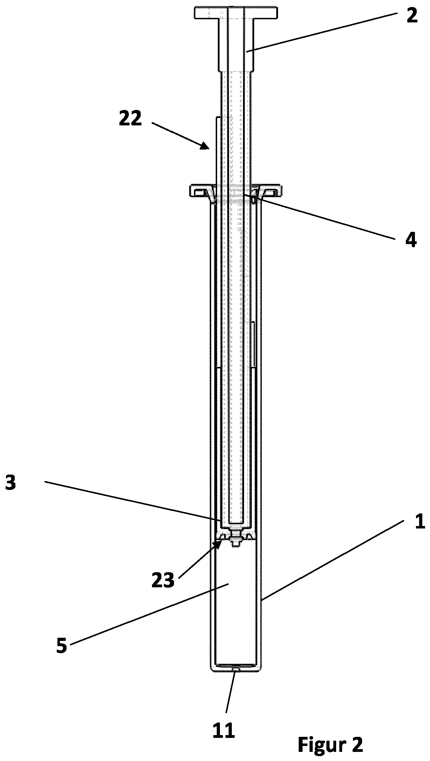

In Figur 2 ist die Dosierpipette nach Figur 1 in einem aufgezogenen Zustand dargestellt. Durch das Herausziehen des Dosierkolben 22 entsteht im Gehäuse 1 zwischen dem proximalen Ende des Gehäuses im Bereich der Ausbringöffnung 11 und einem proximalen Ende 23 des Dosierkolben 22 ein Dosiervolumen 5. Der Dosierkolben 22 kann maximal bis zu einem Anschlag 4 aus dem Gehäuse 1 herausgezogen werden. Somit wird das Dosiervolumen 5 durch ein Zusammenwirken des Dosierkolben 22 und des festen Anschlag 4 begrenzt.

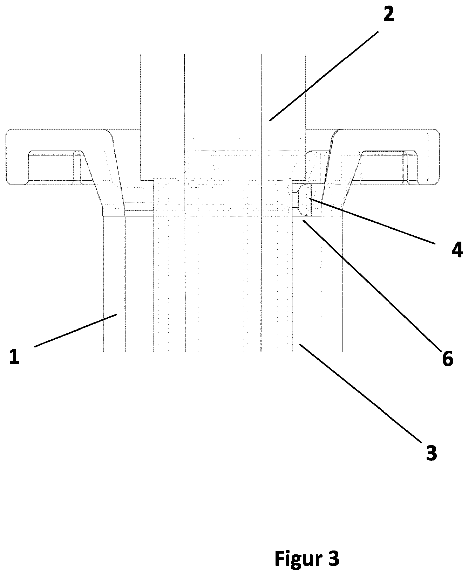

In Figur 3 ist eine Schnittdarstellung gezeigt, die den Bereich der Dosierpipette darstellt, in der sich der feste Anschlag 4 befindet. Der Dosierkolben 22, der aus dem Kolben 2 und dem Dosiereinsatz 3 besteht, kann solange aus dem Gehäuse 1 herausgezogen werden, bis ein Dosieranschlag 6 auf den festen Anschlag 4 trifft und ein weiteres Herausziehen des Dosierkolbens 22 verhindert. Somit treten der Dosieranschlag 6 und der Anschlag 4 in Wirkverbindung und begrenzen das Herausziehen des Dossierkolbens 22.

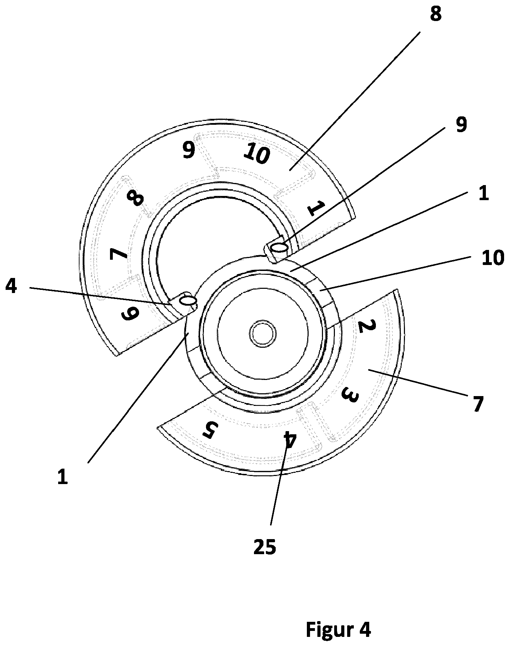

In Figur 4 ist eine Draufsicht auf ein Gehäuse 1 von einer distalen Seite dargestellt. Ein Gehäuserand 7 des Gehäuse 1 ist in dem dargestellten Ausführungsbeispiel zweiteilig ausgeführt. Ein Teil des Gehäuserands 7 ist einstückig mit dem Gehäuse 1 ausgebildet. Der Gehäuserand 7 umfasst weiterhin einen Gehäuseflansch 8, welcher wiederum einstückig mit den festen Anschlägen 4 ausgebildet ist. Der Gehäuseflansch 8 mit den festen Anschlägen 4 wird in einem typischen Ausführungsbeispiel einstückig mit dem Gehäuse 1 bspw. als Spritzgussteil hergestellt.

Für die Aufnahme der Anschläge 4 weist das Gehäuse 1 zumindest eine Ausnehmung 10 auf. Bei einer einstückigen Herstellung des Gehäuses 1 und des Gehäuseflansches 8 weisen diese im Bereich der Anschläge 4 eine Perforation 9 auf, die ein einfaches Abbrechen des Gehäuseflansches 8 vom Gehäuse 1 ermöglichen. Die Anschläge 4 sind in einem Ausführungsbeispiel in Form eines Nockens oder eines Zapfens ausgebildet.

In einem weiteren Ausführungsbeispiel wird der Gehäuseflansch 8 als separates Spritzgussteil hergestellt. Vorteilhafterweise wird der Gehäuseflansch 8 einstückig mit dem Gehäuse 1 als Spritzgussteil hergestellt, wodurch sich die Produktionskosten für das gesamte Gehäuse 1 senken lassen. Beim Zusammensetzen der Dosierpipette wird, nachdem der Dosierkolben 22 in das Gehäuse 1 eingesetzt ist, der Gehäuseflansch 8 mit den Anschlägen 4 in die Aussparungen 10 im Gehäuse 1 gedrückt. Somit ist der Dosierkolben 22 durch die festen Anschläge 4 gegen Herausziehen aus dem Gehäuse 1 gesichert.

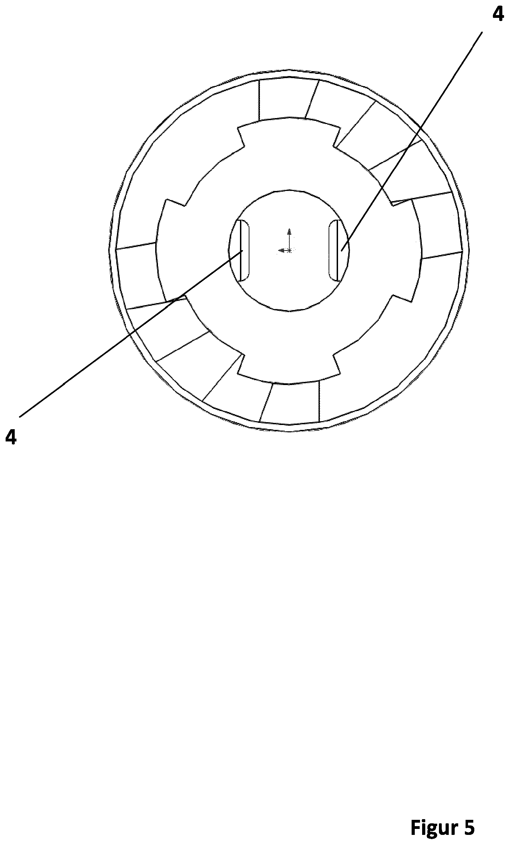

In Figur 5 ist eine Schnittdarstellung durch den Gehäuserand 7 des montierten Gehäuse 1 mit in den Ausnehmungen 10 eingerasteten Anschlägen 4 dargestellt. Mit der Bezeichnung Schnittdarstellung quer durch den Gehäuserand 7 ist eine Schnittebene bezeichnet, die orthogonal zu der Längsschnittebene nach Figur 1 ist.

Durch das Einrasten der festen Anschläge 4 in den Ausnehmungen 10 des Gehäuse 1 findet eine Systemverriegelung statt. Durch diese Systemverriegelung kann verhindert werden, dass der Dosierkolben 22 vollständig aus dem Gehäuse 1 herausgezogen wird. Um eine möglichst sichere Systemverriegelung zu erreichen befinden sich die Ausnehmungen 10 vorzugsweise im Bereich des grössten Durchmessers des Gehäuses 1. Diese Klemmwirkung kann verstärkt werden, wenn sich die Ausnehmungen 10 abseits des grössten Durchmessers des Gehäuses 1 befinden und die festen Anschläge am Gehäuseflansch 8 über den grössten Durchmesser zum Einrasten in die Ausnehmung 10 geschoben werden müssen.

In einem Ausführungsbeispiel kann der Gehäuseflansch 8 nach der Montage wieder entfernt werden, wodurch es ermöglicht wird das der Dosierkolben 22 entnommen werden kann und/oder ausgetauscht werden kann.

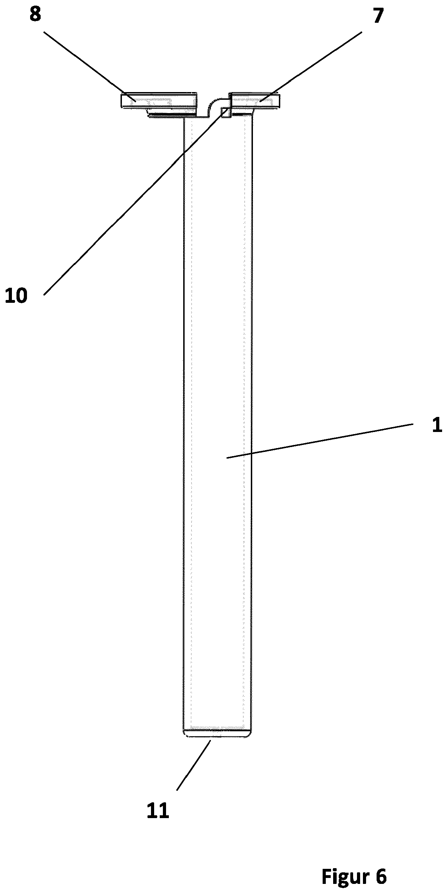

In Figur 6 ist das Herstellungsteil des Gehäuses 1 und des Gehäuseflansches 8 dargestellt, wie es bspw. als Spritzgussteil hergestellt werden kann. Am distalen Ende des Gehäuses ist die Ausnehmung 10, die zur Aufnahme der festen Anschläge 4 vorgesehen ist, zu sehen. Durch Aufschieben des Gehäuseflansches 8 auf das Gehäuse 1 wird, wie bereits zuvor beschrieben, der mehrteilige Gehäuserand 7 geschlossen.

Weiterhin ist auf der proximalen Seite des Gehäuses 1 schematisch die Ausbringöffnung 11 dargestellt. Die Ausbringöffnung 11 ist in einem typischen Ausführungsbeispiel als zylindrischer oder konischer Durchbruch und/oder Bohrung ausgebildet.

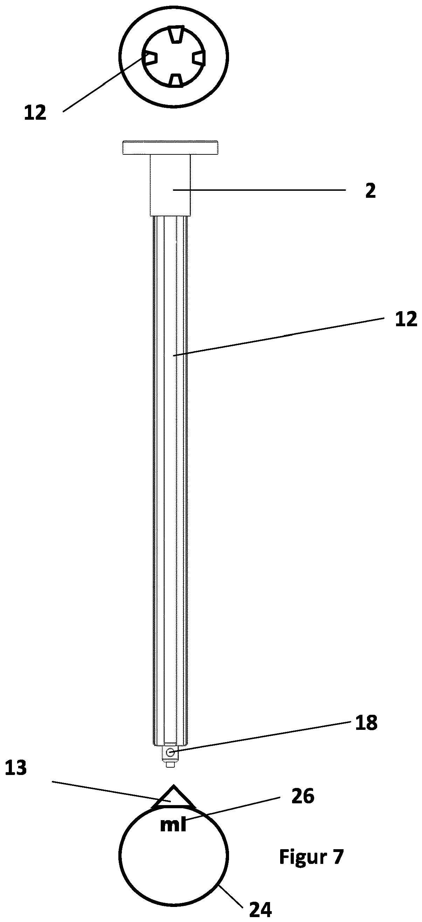

In den Figuren 7a bis 7c sind verschiedene Ansichten des Kolbens 2 des Dosierkolbens 22 dargestellt. In Figur 7b ist dabei eine Seitenansicht auf den Kolben 2 dargestellt. Der Kolben 2 weist zumindest eine, vorzugsweise mehrere Dosiernuten 12 auf, die in einem typischen Ausführungsbeispiel verteilt über den Umfang des Kolbens 2 in diesen eingebracht sind.

Figur 7a zeigt eine Ansicht von der proximalen Seite auf den Kolben 2 mit den Dosiernuten 12. In der Figur 7c ist eine Ansicht auf den Kolben 2 von der distalen Seite dargestellt. Auf der distalen Seite weist der Kolben an einem Kolbenflansch 24 eine Nase 13 auf um den Anwender das Einstellen eines Dosiervolumens 5 in Verbindung mit den Markierungen 25 auf dem Gehäuserand 7 (siehe Figur 4) zu ermöglichen und/oder zu erleichtern.

Weiterhin führt die Nase 13 am Kolbenflansch 24 dazu, dass der Dosierkolben 22 leichter im Gehäuse 1 auf die gewünschte Position verdreht werden kann. Um dies zu ermöglichen ist der Dossierkolben 22 zumindest teilweise verdrehbar in dem Gehäuse 1 der Dosierpipette gelagert. Weiterhin kann auf der Oberseite des Kolbenflansches 24 ein Anwenderhinweis 26 angebracht werden.

Der Kolben 2 weist an seinem proximalen Ende ein Dichtzapfen 21 auf, der vorzugsweise komplementär zu der Ausbringöffnung 11 des Gehäuses 1 ausgebildet ist. Weiterhin weist der Kolben 2 im Bereich des proximalen Endes ein Rastelement 18 auf. Das Rastelement 18 ist dabei komplementär zu einer Ausbuchtung 19 des Dosiereinsatzes 3 ausgebildet.

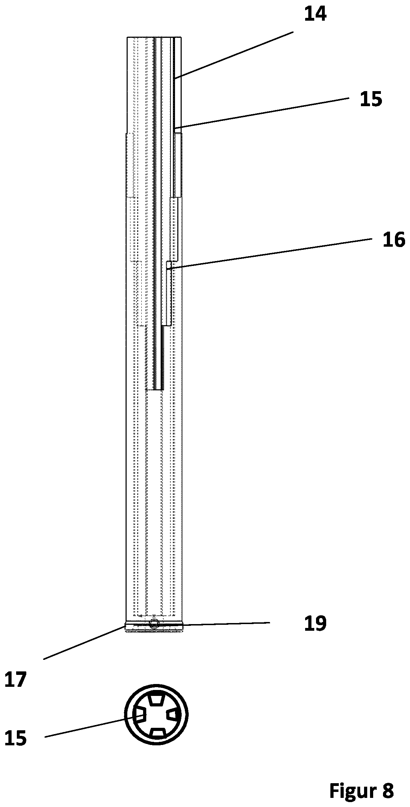

Eine Ausführungsform des Dosiereinsatzes 3 ist in den Figuren 8a und 8b dargestellt. Über den Dosiereinsatz 3 wird nach der Montage mit dem Kolben 2 zumindest ein Dosierweg 14, vorzugsweise mehrere Dosierwege 14, bestimmt. Der Dosierweg 14 erstreckt sich dabei zwischen dem festen Anschlag 4 und einer stufenförmigen Anschlagfläche 15, die mehrere Dosieranschläge 6 aufweist und einer Dosiergeometrie 16 entspricht. Somit kann das genaue Dosiervolumen 5 über die Länge des Dosierwegs 14 bestimmt werden.

In einem typischen Ausführungsbeispiel weist der Dosiereinsatz 3 eine Dosiergeometrie 16 auf, um möglichst verschiedene Dosierwege 14, zu ermöglichen. Die Dosiergeometrie 16 ist hierfür vorzugsweise über den Umfang in dem Dosiereinsatz 3 eingebracht. Der Dosiereinsatz 3 ist dabei so ausgebildet, dass er nachdem er auf den Kolben 2 aufgeschoben wurde, die Dosiernuten 12 auf die Länge der Dosierwege 14 begrenzt.

Um eine formschlüssige Verbindung zwischen dem Kolben 2 und dem Dosiereinsatz 3 zu erreichen, weist der Kolben 2 das Rastelement 18 und der Dosiereinsatz 3 die Ausbuchtung 19 an ihren proximalen Enden auf. Durch das Aufbringen eines Dosiereinsatzes 3, der eine andere Dosiergeometrie 16 aufweist, können mit dem gleichen Kolben 2 und dem gleichen Gehäuse 1 verschiedene Dosierpipetten geschaffen werden, über die verschiedenste Dosiervolumina 5 erreicht werden. Somit ist das Gehäuse 1 dazu geeignet verschiedene Dosierkolben 22 aufzunehmen, wobei insbesondere der Dosiereinsatz 3 unterschiedlich ausgeformt ist. Somit ist eine genaue Dosierung, insbesondere ein genaues und reproduzierbares Dosiervolumen 5 über den Dosiereinsatz einstellbar.

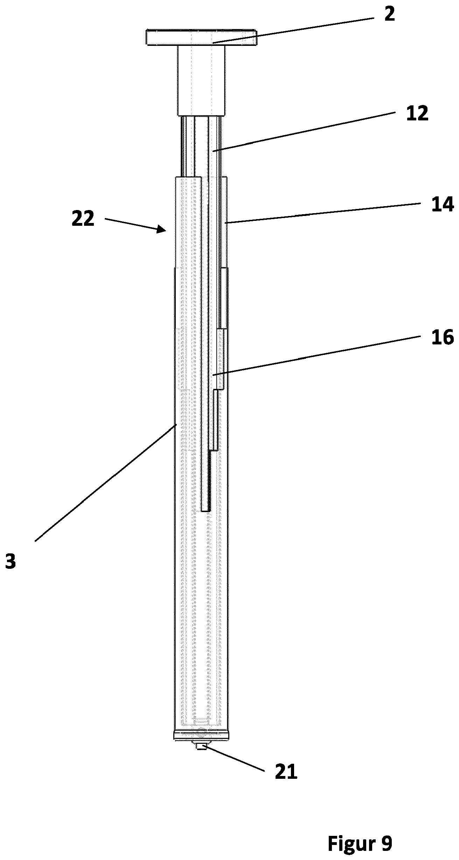

In Figur 9 ist der Dosierkolben 22 im zusammengesetzten Zustand aus Kolben 2 und Dosiereinsatz 3 dargestellt. Dabei ist deutlich zu sehen, dass durch die Dosiergeometrie 16 die Länge der Dosiernuten 12 auf die Länge der Dosierwege 14 begrenzt wird.

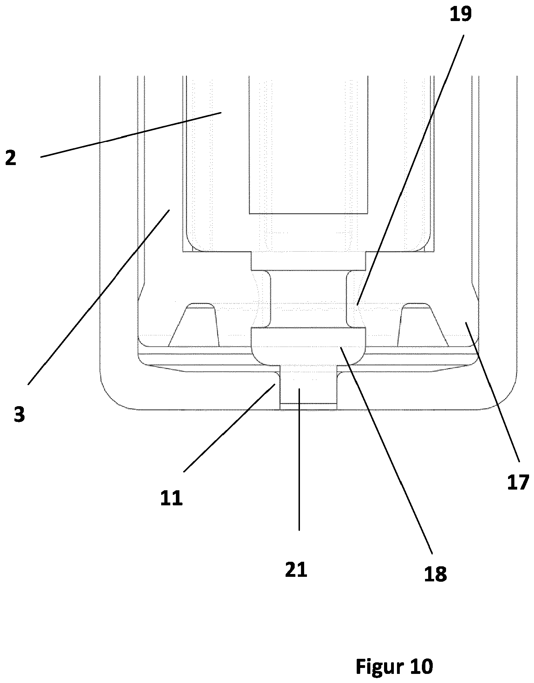

In Figur 10 ist eine Detailschnittdarstellung durch das proximale Ende der Dosierpipette dargestellt. Da das Rastelement 18 des Kolbens 2 die Ausbuchtung 19 des Dosiereinsatzes 3 aufnimmt, entsteht eine formschlüssige Verbindung, die im zusammengesetzten Zustand der Dosierpipette nicht lösbar ist.

Weiterhin ist dargestellt, dass der Dichtzapfen 21 die vorzugsweise zylindrische oder konische Ausbringöffnung 11 des Gehäuses verschliesst und/oder abdichtet. Dadurch ist bspw. eine saubere Applikation eines Medikamentes möglich. Weiterhin ist zu sehen, dass im entleerten Zustand der Dosierpipette fast kein Restvolumen und/oder Todvolumen im Innenraum der Dosierpipette vorhanden ist. Eine Kolbenlippe 17 des Dosiereinsatzes 3 sorgt dafür, dass ein luftfreies und blasenfreies Aufziehen und Entleeren des Mediums über die Dosierpipette ermöglicht wird.

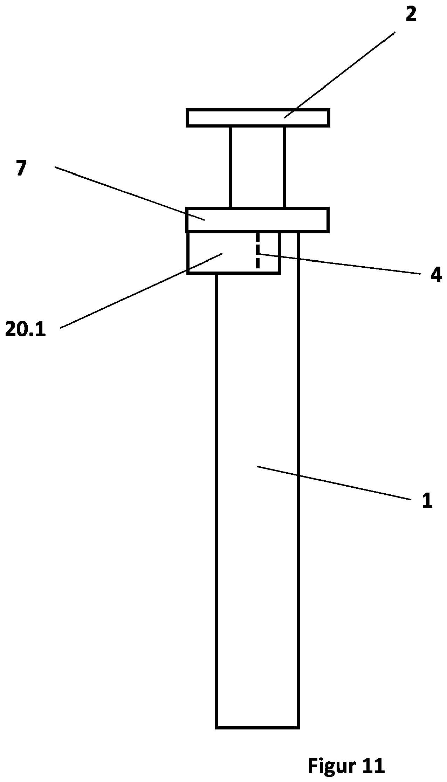

In Figur 11 ist ein weiteres typisches Ausführungsbeispiel einer erfindungsgemässen Dosierpipette dargestellt. Diese weist einen durchgehenden und nicht getrennten Gehäuserand 7 auf. Unterhalb des Gehäuserandes 7 befindet sich in diesem Ausführungsbeispiel ein Verriegelungsring 20.

Da der Verriegelungsring 20 nur einen Teil des Gehäuses 1 umschliesst kann dieser vom Gehäuse 1 entfernt werden, wodurch der Dosierkolben 22 aus dem Gehäuse 1 gezogen werden kann. Dadurch können der Dosierkolben 22 und das Gehäuse 1 gereinigt und/oder sterilisiert werden.

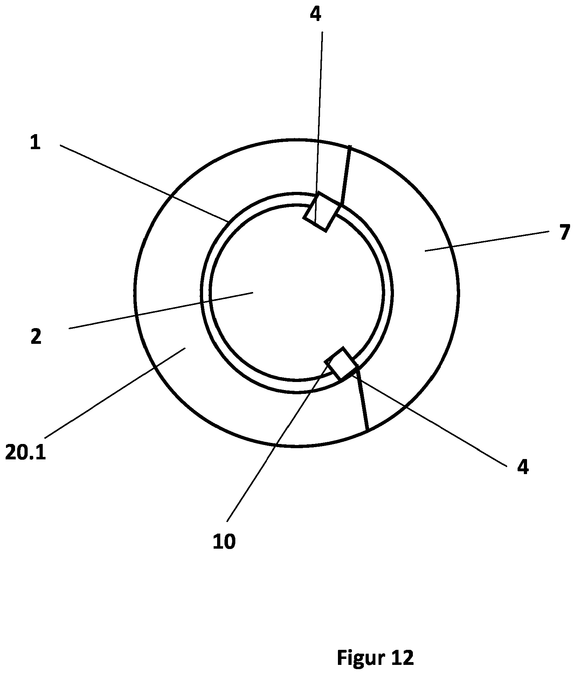

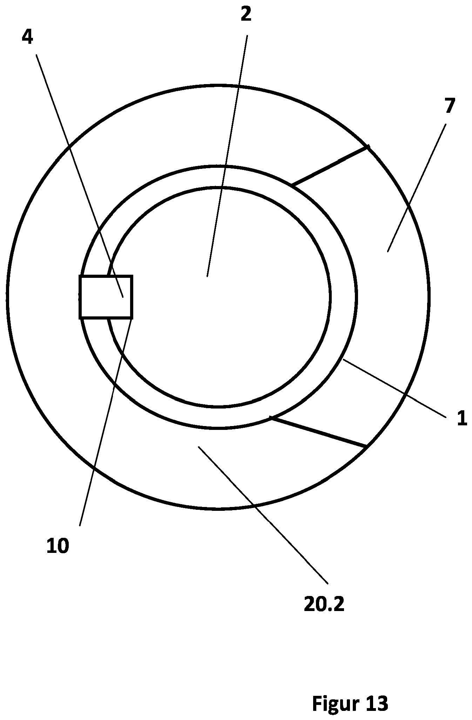

Der Verriegelungsring 20 umschliesst in einem typischen Ausführungsbeispiel ca. ¾ des Gehäuseumfangs des Gehäuse 1. In den Figuren 12 und 13 sind zwei verschiedene Ausführungsformen des Verriegelungsrings 20 dargestellt.

Während in Figur 12 ein Verriegelungsring 20 mit zwei festen Anschlägen 4 dargestellt ist, zeigt Figur 13 einen Verriegelungsring 20 mit einem festen Anschlag 4.

Dadurch, dass der Verriegelungsring 20 mehr als die Hälfte des Gehäuseumfangs des Gehäuse 1 umschliesst, wird verhindert, dass dieser ohne Weiteres einfach vom Gehäuse 1 heruntergeschoben werden kann.

Zur Reinigung der Dosierpipette wird der Verriegelungsring 20 vom Gehäuse 1 heruntergeschoben und anschliessend der Dosierkolben 22 herausgenommen. Nach der Reinigung kann der Dosierkolben 22 wieder in das Gehäuse 1 eingesetzt werden und anschliessend der Verriegelungsring 20 auf das Gehäuse aufgeschoben werden, wobei die festen Anschläge 4 in die Ausnehmung 10 des Gehäuse 1 einrasten müssen.

In einem weiteren nicht dargestellten Ausführungsbeispiel ist der feste Anschlag 4 halbkugelförmig oder als Konus ausgebildet. In diesem Ausführungsbeispiel muss der Verriegelungsring zum Entfernen des Dosierkolbens 22 nicht entfernt werden. Zum Entfernen des Dosierkolbens 22 wird dieser mit erhöhter Zugkraft über den halbkugelförmig oder als Konus ausgebildeten Anschlag 4 gezogen.

Dies wird ermöglicht, da der Anschlag 4 auf Grund der Federwirkung des Verriegelungsrings in diesem Ausführungsbeispiel zumindest teilweise beweglich ist. Nachdem Reinigen kann der Dosierkolben 22 wieder über den Anschlag 4 in das Gehäuse 1 eingeführt werden. Hierfür ist erneut ein höherer Kraftaufwand nötig.

**Bezugszeichenliste**

| | | | | | |
|---|---|---|---|---|---|
| 1 | Gehäuse | 34 | | | |
| 2 | Kolben | 35 | | | |
| 3 | Dosiereinsatz | 36 | | | |
| 4 | Anschlag | 37 | | | |
| 5 | Dosiervolumen | 38 | | | |
| 6 | Dosieranschlag | 39 | | | |
| 7 | Gehäuserand | 40 | | | |
| 8 | Gehäuseflansch | 41 | | | |
| 9 | perforierte Anbindung | 42 | | | |
| 10 | Ausnehmung | 43 | | | |
| 11 | Ausbringöffnung | 44 | | | |
| 12 | Dosiernut | 45 | | | |
| 13 | Nase | 46 | | | |
| 14 | Dosierweg | 47 | | | |
| 15 | Anschlagfläche | 48 | | | |
| 16 | Dosiergeometrie | 49 | | | |
| 17 | Kolbenlippe | 50 | | | |
| 18 | Rastelement | 51 | | | |
| 19 | Ausbuchtung | 52 | | | |
| 20 | Verriegelungsring | 53 | | | |
| 21 | Dichtzapfen | 54 | | | |
| 22 | Dosierkolben | 55 | | | |
| 23 | proximale Seite | 56 | | | |
| 24 | Kolbenflansch | 57 | | | |
| 25 | Markierung | 58 | | | |
| 26 | Anwenderhinweis | 59 | | | |
| 27 | | 60 | | | |
| 28 | | 61 | | | |
| 29 | | 62 | | | |
| 30 | | 63 | | | |
| 31 | | 64 | | | |
| 32 | | 65 | | | |
| 33 | | 66 | | | |

## Patentansprüche

1. Dosierpipettensystem zum Ausbringen eines Mediums, mit:
einem Gehäuse (1),
einer Vielzahl von Dosiereinsätzen (3), und
einem in das Gehäuse (1) einbringbaren Dosierkolben (22), welcher aus einem Kolben (2) und einem frei wählbaren Dosiereinsatz (3) der Vielzahl von Dosiereinsätzen (3) ausgebildet ist,
wobei der Dosierkolben (22) nach dem Einbringen in das Gehäuse (1) mit dem Gehäuse (1) formschlüssig verbunden ist,
wobei ein fester Anschlag (4) einstückig mit einem Gehäuserand (8) des Gehäuses (1) ausgebildet ist und/oder wobei das Dosierpipettensystem einen Verriegelungsring (20) aufweist und ein fester Anschlag (4) einstückig mit dem Verriegelungsring (20) des Dosierpipettensystems ausgebildet ist,
wobei jeder Dosiereinsatz (3) eine stufenförmige Anschlagsfläche (15) mit mehreren Dosieranschlägen (6) aufweist, welche nach dem Einbringen des Dosierkolbens (22) in das Gehäuse (1) mit dem festen Anschlag (4) derart in Wirkverbindung stehen, dass ein Herausziehen des Dosierkolbens (22) aus dem Gehäuse (1) durch den festen Anschlag (4) begrenzt wird, wobei sich zwischen jedem der Dosieranschläge (6) und dem festen Anschlag (4) ein jeweiliger Dosierweg (14) erstreckt, und wobei ein Dosiervolumen (5) von einer Länge des jeweiligen Dosierwegs (14) abhängt,
wobei der Dosierkolben (22) nach dem Einbringen in das Gehäuse (1) im Gehäuse (1) zumindest teilweise verdrehbar gelagert ist, um durch Verdrehen des Dosierkolbens (22) einen der Dosierwege (14) einzustellen, und
wobei die Dosiereinsätze (3) unterschiedlich ausgeformt sind, sodass sich die einstellbaren Dosiervolumina (5) für verschiedene Dosiereinsätze (3) unterscheiden.

2. Dosierpipettensystem nach Anspruch 1, **dadurch gekennzeichnet, dass** eine genaue Dosierung des auszubringenden Mediums über den Dosiereinsatz (3) durch Auswahl des jeweiligen Dosierwegs (14) einstellbar ist.

3. Dosierpipettensystem nach einem der Ansprüche 1 oder 2, **dadurch gekennzeichnet, dass** der Kolben (2) eine Dosiernut (12) aufweist, in welche der feste Anschlag (4) greift.

4. Dosierpipettensystem nach einem der Ansprüche 1 bis 3, **dadurch gekennzeichnet, dass** der Dosiereinsatz (3) eine Ausbuchtung (19) aufweist, die komplementär zu einem Rastelement (18) des Kolbens (2) ausgebildet ist.

5. Dosierpipettensystem nach einem der Ansprüche 1 bis 4, **dadurch gekennzeichnet, dass** der Dosierkolben (22) eine Kolbenlippe (17) aufweist.

6. Dosierpipettensystem nach einem der Ansprüche 1 bis 5, **dadurch gekennzeichnet, dass** der Dosierkolben (22) einen Dichtzapfen (21) aufweist.

## Claims

1. Dosing pipette system for discharging a medium, said dosing pipette system having:
a housing (1),
a plurality of dosing inserts (3), and
a dosing piston (22) that can be introduced into the housing (1) and said dosing piston is embodied from a piston (2) and a freely-selectable dosing insert (3) of the plurality of dosing inserts (3),
wherein after the introduction into the housing (1) the dosing piston (22) is connected in a positive-locking manner to the housing (1),
wherein a fixed stop (4) is embodied as a single piece with a housing edge (8) of the housing (1) and/or wherein the dosing pipette system comprises a locking ring (20) and a fixed stop (4) is embodied as a single piece with the locking ring (20) of the dosing pipette system,
wherein each dosing insert (3) comprises a stepped stop surface (15) having multiple dosing stops (6) that after the introduction of the dosing piston (22) into the housing (1) are in an operative connection with the fixed stop (4) in such a manner that an extraction of the dosing piston (22) from the housing (1) is limited by means of the fixed stop (4), wherein a respective dosing path (14) extends between each of the dosing stops (6) and the fixed stop (4), and wherein a dosing volume (5) depends on a length of the respective dosing path (14),
wherein after the introduction into the housing (1) the dosing piston (22) is mounted in the housing (1) at least in part in a twistable manner in order to set one of the dosing paths (14) by means of twisting the dosing piston (22), and
wherein the dosing inserts (3) are shaped differently so that the dosing volumes (5) that can be set differ for different dosing inserts (3).

2. Dosing pipette system according to claim 1, **characterised in that** a precise dosing of the medium that is to be discharged can be set via the dosing insert (3) by means of selecting the respective dosing path (14).

3. Dosing pipette system according to one of claims 1 or 2, **characterised in that** the piston (2) comprises a dosing groove (12) and the fixed stop (4) grips into said dosing groove.

4. Dosing pipette system according to one of claims 1 to 3, **characterised in that** the dosing insert (3) comprises a bulge (19) that is embodied in a complementary manner with respect to a latching element (18) of the piston (2).

5. Dosing pipette system according to one of claims 1 to 4, **characterised in that** the dosing piston (22) comprises a piston lip (17).

6. Dosing pipette system according to one of claims 1 to 5, **characterised in that** the dosing piston (22) comprises a sealing pin (21).

## Revendications

1. Système de pipette de dosage pour distribuer un milieu, comprenant :
un boîtier (1),
une pluralité d'inserts de dosage (3) et
un piston de dosage (22) pouvant être introduit dans le boîtier (1), lequel est formé d'un piston (2) et d'un insert de dosage (3) pouvant être librement choisi parmi la pluralité d'inserts de dosage (3),
le piston de dosage (22) étant relié par complémentarité de forme au boîtier (1) après son introduction dans le boîtier (1),
une butée fixe (4) étant réalisée d'un seul tenant avec un bord de boîtier (8) du boîtier (1) et/ou le système de pipette de dosage présentant une bague de verrouillage (20) et une butée fixe (4) étant réalisée d'un seul tenant avec la bague de verrouillage (20) du système de pipette de dosage,
chaque insert de dosage (3) présentant une surface de butée étagée (15) avec plusieurs butées de dosage (6) qui, après l'introduction du piston de dosage (22) dans le boîtier (1), sont en liaison fonctionnelle avec la butée fixe (4) de telle sorte qu'une extraction du piston de dosage (22) du boîtier (1) est limitée par la butée fixe (4), un trajet de dosage respectif (14) s'étendant entre chacune des butées de dosage (6) et la butée fixe (4), et un volume de dosage (5) dépendant d'une longueur du trajet de dosage respectif (14),
le piston de dosage (22), après avoir été introduit dans le boîtier (1), étant monté dans le boîtier (1) de manière à pouvoir tourner au moins partiellement afin de régler l'un des trajets de dosage (14) par rotation du piston de dosage (22), et
les inserts de dosage (3) étant formés différemment, de sorte que les volumes de dosage (5) réglables diffèrent pour différents inserts de dosage (3).

2. Système de pipette de dosage selon la revendication 1, **caractérisé en ce qu'**un dosage précis du milieu à distribuer peut être réglé au moyen de l'insert de dosage (3) en sélectionnant le trajet de dosage respectif (14).

3. Système de pipette de dosage selon l'une des revendications 1 ou 2, **caractérisé en ce que** le piston (2) présente une rainure de dosage (12) dans laquelle s'engage la butée fixe (4).

4. Système de pipette de dosage selon l'une des revendications 1 à 3, **caractérisé en ce que** l'insert de dosage (3) présente un renflement (19) qui est complémentaire d'un élément d'arrêt (18) du piston (2).

5. Système de pipette de dosage selon l'une des revendications 1 à 4, **caractérisé en ce que** le piston de dosage (22) présente une lèvre de piston (17).

6. Système de pipette de dosage selon l'une des revendications 1 à 5, **caractérisé en ce que** le piston de dosage (22) présente un bouchon d'étanchéité (21).
